# FASCICULE DE BREVET EUROPEEN

(11) **EP 4 436 641 B1**
(45) Date de publication et mention de la délivrance du brevet: **21.01.2026**
(21) Numéro de dépôt: 22826142.6
(22) Date de dépôt: 22.11.2022
(51) Int. Cl.: A61M 15/08, A61M 11/00

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 24.11.2021 FR 2112457
(43) Date de publication de la demande: 02.10.2024
(73) Titulaire: APTAR France SAS, 27110 Le Neubourg (FR)
(72) Inventeur: BAILLET, Matthieu, 76000 ROUEN (FR)
(74) Mandataire: CAPRI
(86) Numéro de dépôt international: PCT/FR2022/052150
(87) Numéro de publication internationale: WO 2023/094760

(56) Documents cités:
- FR-A1- 3 115 210
- US-A1- 2010 145 275
- US-A1- 2016 068 326
- US-B2- 7 681 570

## Description

La présente invention concerne un dispositif de distribution de produit fluide de type bidose, en particulier pour une pulvérisation nasale.

Par dispositif de distribution de type bidose, on entend un dispositif contenant deux doses de produit fluide à distribuer lors de deux actionnements successifs du dispositif de distribution.

Les dispositifs de type bidose sont bien connus dans l'état de la technique. Ces dispositifs comportent généralement un réservoir contenant les deux doses de produit fluide à distribuer, et un organe de distribution, qui est généralement un piston, qui est monté coulissant dans ledit réservoir, et qui est déplacé pour distribuer le produit fluide contenu dans ledit réservoir. Le déplacement du piston se fait en deux courses d'actionnement successives, de sorte qu'une première dose est distribuée lors d'un premier actionnement et une seconde dose est distribuée lors d'un deuxième actionnement.

Avec ce type de dispositif de type bidose, il y a des moyens de séparation de dose, pour définir les deux doses, et généralement aussi des moyens d'accumulation d'énergie, prévus pour imposer l'application d'une certaine force pour pouvoir actionner le dispositif. Ces moyens d'accumulation d'énergie permettent d'empêcher des actionnements non souhaités ou accidentels, par exemple pendant le stockage ou le transport. Ils garantissent également une distribution de la totalité de la dose à chaque actionnement en générant une précompression dans la main de l'utilisateur au moment de l'actionnement. Ces moyens d'accumulation d'énergie, qui peuvent par exemple être formés par des éléments déformables, déplaçables ou cassables, permettent aussi de prédéfinir la force nécessaire à l'actionnement, et donc les paramètres du spray généré en sortie du dispositif.

Or, lors de l'assemblage et/ou l'actionnement du dispositif, il y a un risque d'altération desdits moyens d'accumulation d'énergie. Ainsi, lors de l'assemblage, certains éléments formant ces moyens d'accumulation d'énergie peuvent subir des chocs susceptibles de les modifier, avec par conséquent un impact possible sur les performances d'accumulation d'énergie. Il s'en suit un risque d'augmentation de la variabilité de l'effort d'actionnement requis pour surmonter les moyens d'accumulation d'énergie, et donc une variabilité dans les propriétés du spray lors de la distribution de dose. Or, une telle variabilité n'est pas souhaitable, notamment en termes de répétabilité et stabilité des performances de distribution des doses. Un risque similaire existe lors de l'actionnement de la première dose pour les moyens d'accumulation d'énergie prévus pour la seconde dose, et qui peuvent lors de ce premier actionnement subir des chocs susceptibles de les altérer.

Les documents US20100145275A1, US20160068326A1 et US7681570B2 et FR3115210A1 décrivent des dispositifs de l'état de la technique.

La présente invention a pour but de fournir un dispositif de distribution de produit fluide qui ne reproduit pas les inconvénients susmentionnés.

La présente invention a ainsi pour but de fournir un dispositif de distribution de produit fluide qui garantit l'intégrité des moyens d'accumulation d'énergie avant chaque utilisation.

La présente invention a également pour but de fournir un tel dispositif de distribution de produit fluide qui réduit la variabilité des efforts d'actionnement et améliore la répétabilité et la stabilité des paramètres de distribution de chaque dose.

La présente invention a également pour but de fournir un tel dispositif de distribution de produit fluide qui soit simple et peu coûteux à fabriquer et à assembler.

La présente invention a donc pour objet un dispositif de distribution de produit fluide comportant un réservoir contenant deux doses de produit fluide, un organe de distribution, tel qu'un piston, monté coulissant dans ledit réservoir pour distribuer du produit fluide, un corps principal, un corps central solidaire dudit corps principal, un organe de support recevant ledit réservoir, une tête de distribution pourvue d'un orifice de distribution et un organe d'actionnement axialement déplaçable à l'intérieur dudit corps principal et coopérant avec ledit organe de support pour réaliser les actionnements successifs du dispositif en déplaçant ledit organe de support par rapport audit corps central pour ainsi déplacer ledit organe d'actionnement dans ledit réservoir et ainsi distribuer du produit fluide à travers ledit orifice de distribution, un ressort de rappel étant prévu pour ramener ledit organe d'actionnement dans sa position de départ après chaque actionnement, ledit dispositif comportant des moyens d'accumulation d'énergie comportant des premier et second éléments de poussée formés sur ledit corps central et des premier et second éléments de résistance formés sur ledit organe de support, ledit premier élément de poussée étant adapté à coopérer avec ledit premier élément de résistance pour accumuler de l'énergie lors de la distribution de la première dose, et ledit second élément de poussée étant adapté à coopérer avec ledit second élément de résistance pour accumuler de l'énergie lors de la distribution de la seconde dose, chaque élément de poussée comportant une patte radialement déformable, pourvue à son extrémité axiale inférieure d'une zone de poussée centrale, dont la surface axialement inférieure vient en contact d'un élément de résistance respectif lors des actionnements, dans lequel:
- ledit organe de support comporte deux profils longitudinaux diamétralement opposés, chaque profil longitudinal comportant, en partant du bord axial supérieur : un premier profil de déformation, une première fenêtre, un second profil de déformation, et une seconde fenêtre,
- la première fenêtre d'un profil longitudinal comporte ledit premier élément de résistance, et la seconde fenêtre de l'autre profil longitudinal comporte ledit second élément de résistance,
- chaque profil de déformation comporte : une rampe centrale s'étendant axialement et inclinée radialement vers l'extérieur en direction du bas, disposée entre des première et seconde nervures latérales axialement droites et de dimension radiale constante,
- chaque zone de poussée centrale est disposée entre une première et une seconde zone inclinée,
- de telle sorte que lorsque ledit organe de support se déplace axialement par rapport audit corps central lors de l'assemblage, chaque élément de poussée coopère avec un premier profil de déformation respectif de telle sorte que chaque zone de poussée centrale n'entre pas en contact avec ledit organe de support.

Avantageusement, lorsque ledit organe de support se déplace axialement par rapport audit corps central lors de la distribution de la première dose, ledit second élément de poussée coopère avec un second profil de déformation de telle sorte que sa zone de poussée centrale n'entre pas en contact avec ledit organe de support.

Avantageusement, lesdits premier et second éléments de résistance sont décalés axialement sur ledit corps de support.

Avantageusement, lesdits premier et second éléments de poussée sont disposés diamétralement opposés sur ledit corps central.

Avantageusement, lesdits éléments de résistance sont formés par des ponts sécables, qui se cassent lors de l'actionnement, ou par des poutres déformables, qui se déforment lors de l'actionnement.

Avantageusement, lesdits premier et second éléments de poussée sont identiques, ledit premier élément de poussée coopérant avec un profil longitudinal et ledit second élément de poussée coopérant avec l'autre profil longitudinal.

Avantageusement, lesdits premiers profils de déformation coopèrent avec les deux éléments de poussée lors de l'assemblage, et lesdits seconds profils de déformation coopèrent avec lesdits deux éléments de poussée lors de la distribution de la première dose.

Avantageusement, la surface radialement interne de chaque élément de poussée a une forme de T.

Avantageusement, lors de l'assemblage, lesdites zones de poussée vont chacune coopérer avec une rampe centrale respective d'un premier profil de déformation, pendant que lesdites zones inclinées vont coopérer avec lesdites nervures latérales.

Avantageusement, lors de la distribution de la première dose, lesdites zones de poussée vont chacune coopérer avec une rampe centrale respective d'un second profil de déformation, pendant que lesdites zones inclinées vont coopérer avec lesdites nervures latérales.

Avantageusement, lors de l'assemblage, lesdites zones inclinées de chaque élément de poussée glissent sur le bord axial supérieur desdites nervures latérales dudit premier profil de déformation, ce qui déforme lesdites pattes déformables radialement vers l'extérieur, de sorte que chaque zone de poussée évite tout contact avec ledit organe de support, en étant d'abord face à la partie de plus petit diamètre de chaque rampe centrale, puis en s'écartant radialement vers l'extérieur au fur et à mesure de l'assemblage.

Avantageusement, lors de la distribution de la première dose, lesdites zones inclinées de chaque élément de poussée glissent sur le bord axial supérieur desdites nervures latérales dudit second profil de déformation, ce qui déforme lesdites pattes déformables radialement vers l'extérieur, de sorte que chaque zone de poussée évite tout contact avec ledit organe de support, en étant d'abord face à la partie de plus petit diamètre de chaque rampe centrale, puis en s'écartant radialement vers l'extérieur au fur et à mesure de la distribution de la première dose.

Avantageusement, en fin d'assemblage, lesdites pattes déformables s'encliquètent dans une première fenêtre respective, ledit premier élément de poussée étant disposé face audit premier élément de résistance, pour réaliser l'accumulation d'énergie lors de la distribution de la première dose.

Avantageusement, en fin de distribution de la première dose, lesdites pattes déformables s'encliquètent dans une seconde fenêtre respective, ledit second élément de poussée étant disposé face audit second élément de résistance, pour réaliser l'accumulation d'énergie lors de la distribution de la seconde dose.

Ces avantages et caractéristiques et d'autres de la présente invention apparaitront plus clairement au cours de la description détaillée suivante, faite en référence aux dessins joints, donnés à titre d'exemples non limitatifs, et sur lesquels
Les figures 1 à 4 sont des vues schématiques en section transversale d'un dispositif de distribution de produit fluide selon un premier mode de réalisation avantageux, respectivement avant distribution de la première dose, après distribution de la première dose, avant distribution de la seconde dose et après distribution de la seconde dose,
La figure 5 est une vue schématique en perspective de côté du corps principal du dispositif des figures 1 à 4,
La figure 6 est une vue schématique en perspective de dessous du corps principal de la figure 5,
La figure 7 est une vue schématique partielle en perspective d'un premier élément de poussée solidaire du corps principal des figures 5 et 6,
Les figures 8 et 9 sont des vues schématiques de côté du corps de support du dispositif des figures 1 à 4,
La figure 10 est une vue schématique lors de l'assemblage du corps de support dans le corps principal,
La figure 11 est une vue de détail agrandie de la figure 10,
Les figures 12 à 15 sont des vues de détails agrandies montrant différentes positions de l'élément de poussée pendant l'assemblage,
Les figures 16 à 19 sont des vues schématiques en section transversale d'un dispositif de distribution de produit fluide selon un second mode de réalisation avantageux, respectivement avant distribution de la première dose, après distribution de la première dose, avant distribution de la seconde dose et après distribution de la seconde dose,
La figure 20 est une vue schématique en perspective de dessous du corps central du dispositif des figures 1 à 4,
La figure 21 est une vue schématique partielle en perspective d'un premier élément de poussée solidaire du corps principal de la figure 20,
Les figures 22 et 23 sont des vues schématiques de côté du corps de support du dispositif des figures 16 à 19,
La figure 24 est une vue schématique lors de l'assemblage du corps de support dans le corps central,
La figure 25 est une vue de détail agrandie de la figure 24, et
Les figures 26 à 29 sont des vues de détails agrandies montrant différentes positions de l'élément de poussée pendant l'assemblage.

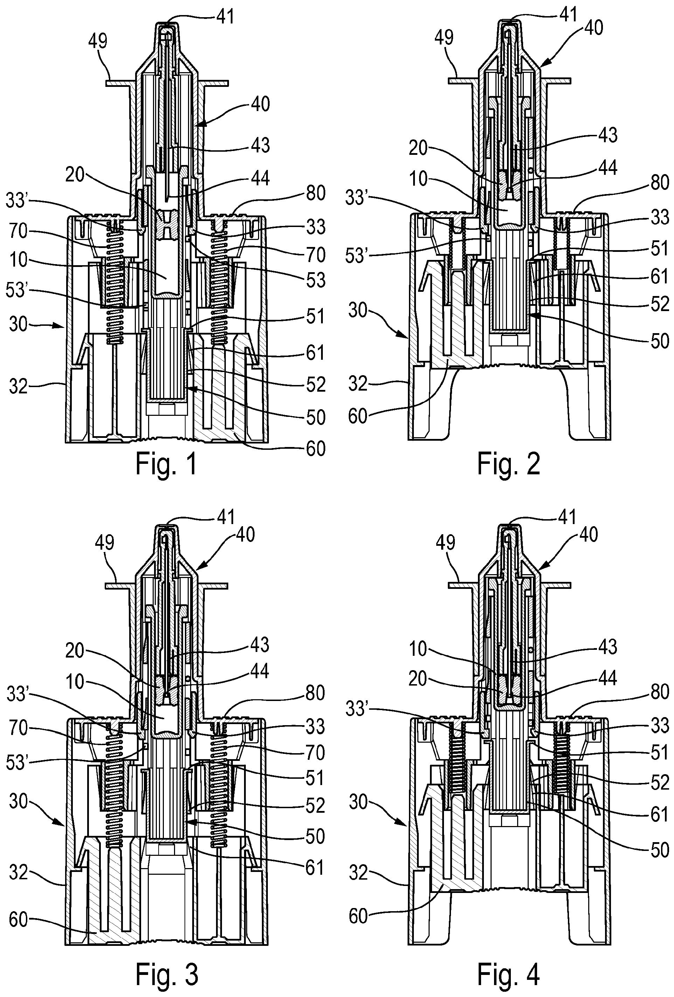

Dans la description ci-après, les termes "axial" et "radial" se réfèrent à l'axe central longitudinal du dispositif. Les termes "haut", "bas", " supérieur" et "inférieur" se réfèrent à la position droite représentée sur les figures 1 à 4 et 16 à 19.

La présente invention sera décrite ci-après en référence à deux modes de réalisation d'un bidose, c'est-à-dire un dispositif contenant deux doses de produit fluide à distribuer lors de deux actionnements successifs du dispositif. Il est toutefois entendu que les dispositifs de type bidose représentés sur les dessins ne sont que des exemples de réalisation possibles auquel la présente invention s'applique, et il est entendu que la présente invention s'applique de manière plus générale à tout type de dispositif contenant deux doses.

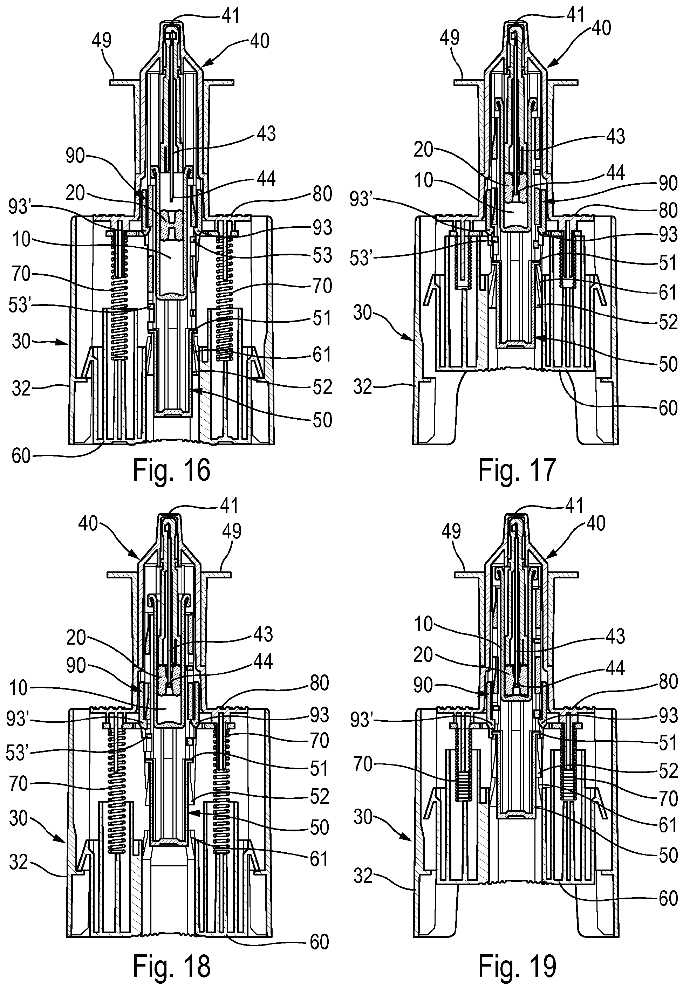

En se référant aux figures, le dispositif de distribution est un bidose qui comporte un réservoir 10 contenant deux doses de produit fluide. Un organe de distribution, tel qu'un piston 20, est monté coulissant dans ledit réservoir 10. Dans la position avant actionnement du dispositif, représentée sur les figures 1 et 16, ledit piston 20 fait office de bouchon en isolant le contenu du réservoir 10.

Un corps principal 30 est assemblé sur ledit réservoir 10 en étant déplaçable axialement par rapport à celui-ci. En particulier, un déplacement axial du corps principal 30 par rapport au réservoir 10 provoque le déplacement du piston 20 dans le réservoir 10 et ainsi la distribution du produit fluide contenu dans ledit réservoir.

Une tête de distribution 40 est assemblée sur le corps principal 30. La tête de distribution 40 comporte un canal de distribution 43 qui mène depuis une pointe de percement 44 jusqu'à un orifice de distribution 41 de la tête de distribution 40. En amont de l'orifice de distribution 41, il peut être prévu un profil de pulvérisation, qui peut être d'un quelconque type connu et qui n'est pas représenté plus en détail sur les dessins, pour distribuer le produit fluide sous forme de spray. Un organe d'appui nasal 49 peut être disposé autour de la tête de distribution 40, pour limiter l'insertion de ladite tête de distribution 40 dans une narine et/ou orienter cette insertion.

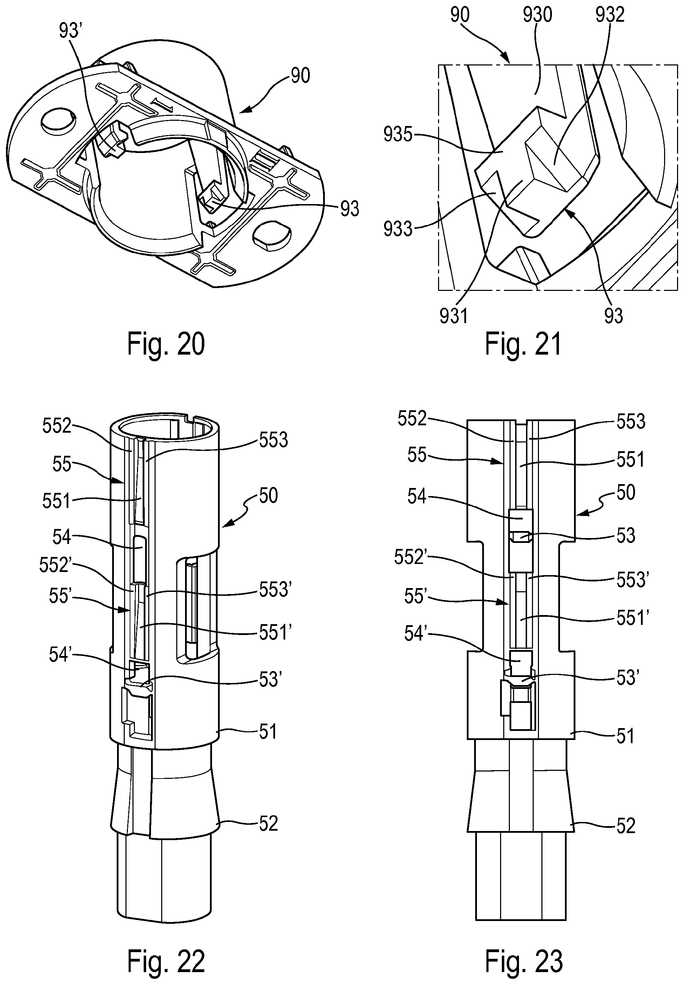

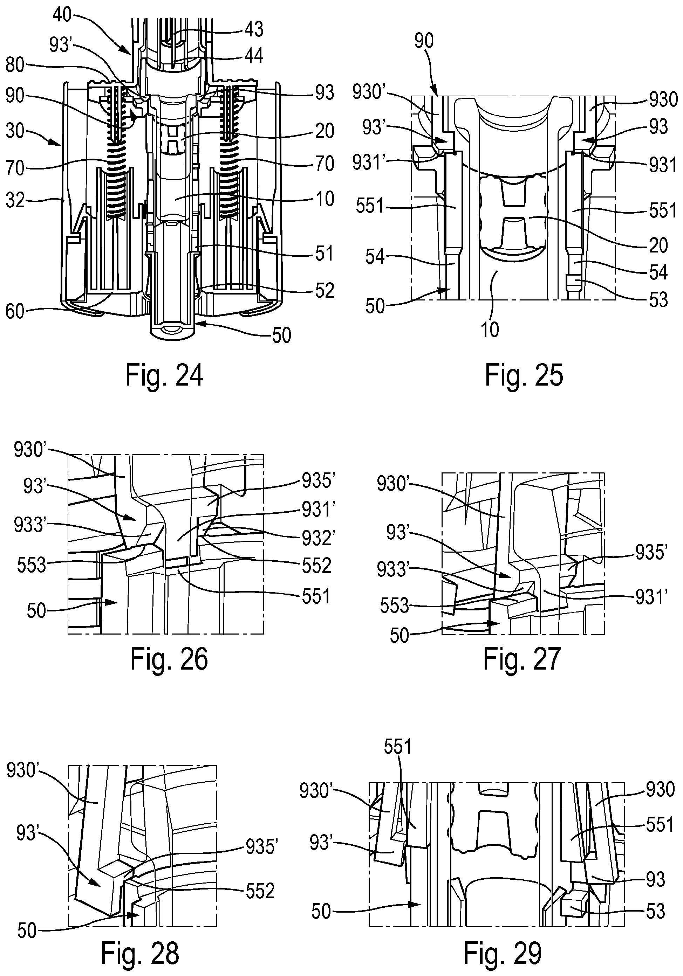

Dans le premier mode de réalisation des figures 1 à 15, la tête de distribution 40 est assemblée fixement sur le corps principal 30, alors que dans le second mode de réalisation des figures 16 à 29, la tête de distribution 40 et le corps principal 30 sont formées d'une seule pièce monobloc.

Un corps central 90 est prévu dans le corps principal 30, ledit corps central 90 incorporant des moyens d'accumulation d'énergie, comme cela sera décrit plus en détails ci-après. Dans le premier mode de réalisation des figures 1 à 15, le corps central 90 est réalisé d'une pièce monobloc avec le corps central 30, alors que dans le second mode de réalisation des figures 16 à 29, le corps central 90 est fixé au corps central 30.

Le réservoir 10 est fixé dans un corps de support 50, qui est donc solidaire dudit réservoir 10, et qui se déplace ensemble avec lui.

Le corps principal 30 comporte une jupe latérale inférieure 32 adaptée à coopérer avec un organe d'actionnement 60. Un élément repose-doigts 80 est formé sur le corps principal 30 et/ou sur la tête de distribution 40, ou en variante il peut être assemblé sur ledit corps principal 30 et/ou sur la tête de distribution 40.

Ledit organe d'actionnement 60 est axialement déplaçable à l'intérieur de ladite jupe latérale 32 du corps principal 30 pour réaliser les actionnements successifs de dispositif. L'organe d'actionnement 60 comporte au moins une patte inclinée 61 qui est adaptée à coopérer avec des projections 51, 52 du corps de support 50 pour réaliser les actionnements successifs.

Au moins un ressort de rappel 70 est monté entre l'organe d'actionnement 60 et le corps principal 30 et/ou la tête de distribution 40, pour ramener ledit organe d'actionnement 60 dans sa position de départ après chaque actionnement. Dans les exemples représentés sur les figures, il y a deux ressorts 70 parallèles, mais d'autres mises en œuvre avec un nombre quelconque de ressorts sont possibles.

Le fonctionnement du dispositif représenté sur les figures est le suivant. Dans la position de repos des figures 1 et 16, le piston bouchon 20 isole le contenu du réservoir 10 de l'atmosphère. Lorsque l'utilisateur appuie simultanément sur le repose-doigt 80 et sur l'organe d'actionnement 60, il va déplacer ledit organe d'actionnement 60 vers le haut à l'intérieur de la jupe latérale 32 du corps principal 30. Ceci va pousser le corps de support 50 axialement vers le haut, par l'intermédiaire des pattes 61 qui vont pousser sur l'épaulement 51 dudit corps de support 50. Ceci va comprimer le ressort 70 et déplacer le réservoir 10 par rapport au corps principal 30. Lorsque le réservoir 10 va commencer à se déplacer par rapport au corps principal 30, l'extrémité de percement 44 du canal de distribution 43 va venir percer le piston de bouchon 20 pour faire communiquer l'intérieur du réservoir 10 avec ledit canal d'expulsion 43. Une poursuite de l'actionnement va provoquer un déplacement du piston 20 à l'intérieur du réservoir 10 et donc une distribution de la première dose. Le produit fluide est donc poussé par ledit piston 20 à travers l'extrémité de percement 44 dans le canal de distribution 43, puis hors du dispositif à travers l'orifice de distribution 41.

Après distribution de la première dose, le dispositif est dans la position représentée sur les figures 2 et 17, et lorsque l'utilisateur relâche l'organe d'actionnement 60, le ressort 70 va ramener celui-ci vers sa position de départ. Pendant ce retour de l'organe d'actionnement 60, le réservoir et le corps de support 50 ne reviennent pas en arrière, car ces deux composants restent maintenus dans le corps principal 30. Avantageusement, des moyens antiretour sont prévus pour empêcher un retour dudit corps de support 50 et/ou dudit réservoir 10. Lorsque l'organe d'actionnement 60 revient sous l'effet du ressort de rappel 70 vers sa position de repos, les pattes 61 vont se positionner sous la deuxième projection 52 du corps de support 50, ce qui va permettre à l'utilisateur d'actionner une deuxième fois le dispositif pour distribuer la deuxième dose de produit fluide.

Les figures 3, 4 et 18, 19 représentent respectivement les positions avant et après distribution de la deuxième dose.

Le dispositif comporte des moyens d'accumulation d'énergie. Des premiers moyens d'accumulation d'énergie sont prévus pour la première dose et des seconds moyens d'accumulation d'énergie sont prévus pour la seconde dose.

Dans le premier mode de réalisation, représenté sur les figures 1 à 15, le corps central 90 est réalisé d'une pièce monobloc avec le corps central 30, et la tête de distribution 40 est fixée sur ledit corps central 30.

Dans le second mode de réalisation représenté sur les figures 16 à 29, la tête de distribution 40 est réalisée d'une pièce monobloc avec le corps central 30, et le corps central 90 est fixé sur ledit corps central 30.

Les premiers moyens d'accumulation d'énergie comportent un premier élément de résistance 53, formé sur le corps de support 50, coopérant avec un premier élément de poussée correspondant 33, 93 formé sur le corps central 90.

Les seconds moyens d'accumulation d'énergie comportent un second élément de résistance 53', formé sur le corps de support 50, coopérant avec un second élément de poussée correspondant 33', 93' formé sur le corps central 90.

Les premier et second éléments de résistance 53, 53' sont décalés axialement sur le corps de support 50.

Les premier et second éléments de poussée 33, 33', respectivement 93, 93', sont disposés diamétralement opposés sur le corps central 90.

L'organe de support 50 comporte deux profils longitudinaux diamétralement opposés, chaque profil longitudinal comportant, en partant du bord axial supérieur : un premier profil de déformation 55, une première fenêtre 54, un second profil de déformation 55', sensiblement identique au premier profil de déformation 55, et une seconde fenêtre 54'.

Chaque profil longitudinal ne comporte qu'un seul élément de résistance 53, 53', prévu dans une seule de ses fenêtres 54, 54'. Ainsi, si un premier profil longitudinal comporte le premier élément de résistance 53 dans sa première fenêtre 54, alors sa seconde fenêtre est dépourvue d'élément de résistance. Dans ce cas, l'autre profil longitudinal comporte le second élément de résistance 53' dans sa seconde fenêtre 54', et sa première fenêtre 54 est dépourvue d'élément de résistance.

Les éléments de résistance 53, 53' peuvent être formés par des ponts sécables, qui se cassent lors de l'actionnement, ou par des poutres déformables, qui se déforment lors de l'actionnement.

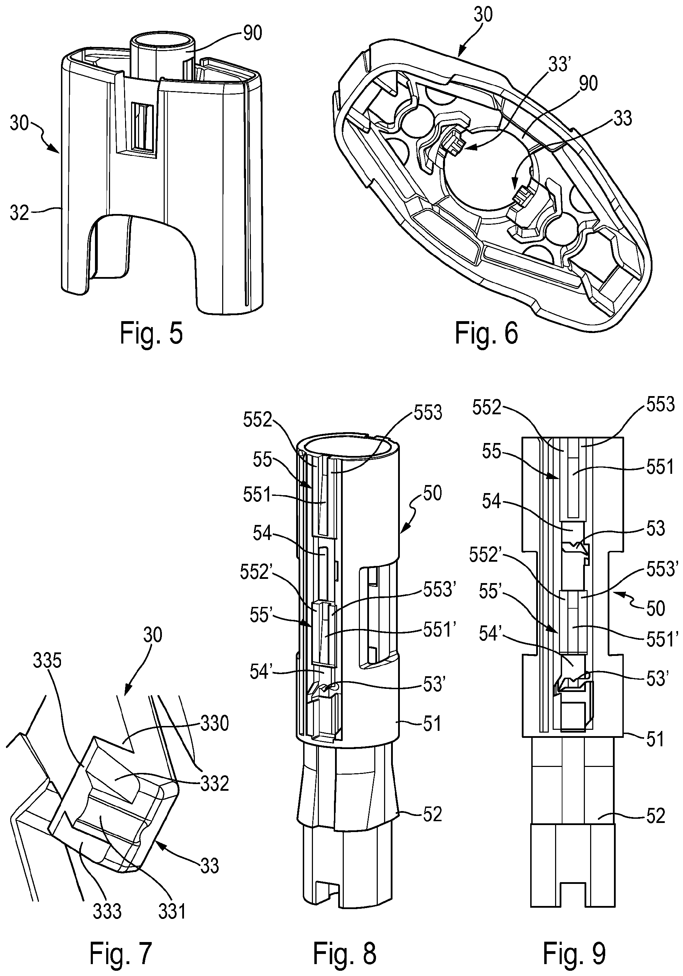

Les premier et second profils de déformation 55, 55' de chaque profil longitudinal sont avantageusement identiques, et comportent chacun: une rampe centrale 551, 551' s'étendant axialement et inclinée radialement vers l'extérieur en direction du bas, disposée entre des première et seconde nervures latérales 552, 552' et 553, 553' axialement droites et de dimension radiale constante. Ceci est particulièrement visible sur la figure 8.

Les premier et second éléments de poussée 33, 93 et 33', 93' sont identiques, le premier élément de poussée 33, 93 coopérant avec un profil longitudinal et le second élément de poussée 33', 93' coopérant avec l'autre profil longitudinal.

Les éléments de poussée étant identiques dans les deux modes de réalisation, la description ci-après sera faite en référence au premier mode de réalisation des figures 1 à 15, avec les éléments de poussée 33, 33', mais il est entendu que cette description s'applique de la même manière au second mode de réalisation des figures 16 à 29, avec les éléments de poussée 93, 93'.

Chaque élément de poussée 33, 33' comporte une patte radialement déformable 330, 330', pourvue à son extrémité axiale inférieure d'une zone de poussée 331, 331' centrale, dont la surface axialement inférieure vient en contact d'un élément de résistance respectif 53, 53' lors des actionnements.

Ainsi, pour garantir l'intégrité de ces deux zones de poussée 331, 331' lors de l'assemblage, il est souhaitable qu'elles ne subissent aucun choc lors de l'assemblage. De même, pour garantir l'intégrité de la seconde zone de poussée 331' lors de la distribution de la première dose, il est souhaitable qu'elle ne subisse aucun choc lors de cette première distribution. Les premiers profils de déformation 55 coopèrent avec les deux éléments de poussée 33, 33' lors de l'assemblage, et les seconds profils de déformation 55' coopèrent avec les deux éléments de poussée 33, 33' lors de la distribution de la première dose.

Chaque zone de poussée 331, 331' est disposée entre une première et une seconde zone inclinée 332, 332' et 333, 333', comme visible sur la figure 7. Ainsi, la surface radialement interne 335 de chaque élément de poussée 33, 33' a avantageusement une forme de T.

Lors de l'assemblage, les zones de poussée 331, 331' vont chacune coopérer avec une rampe centrale respective 551, pendant que les zones inclinées 332, 333 et 332', 333' vont coopérer avec les nervures latérales 552, 553.

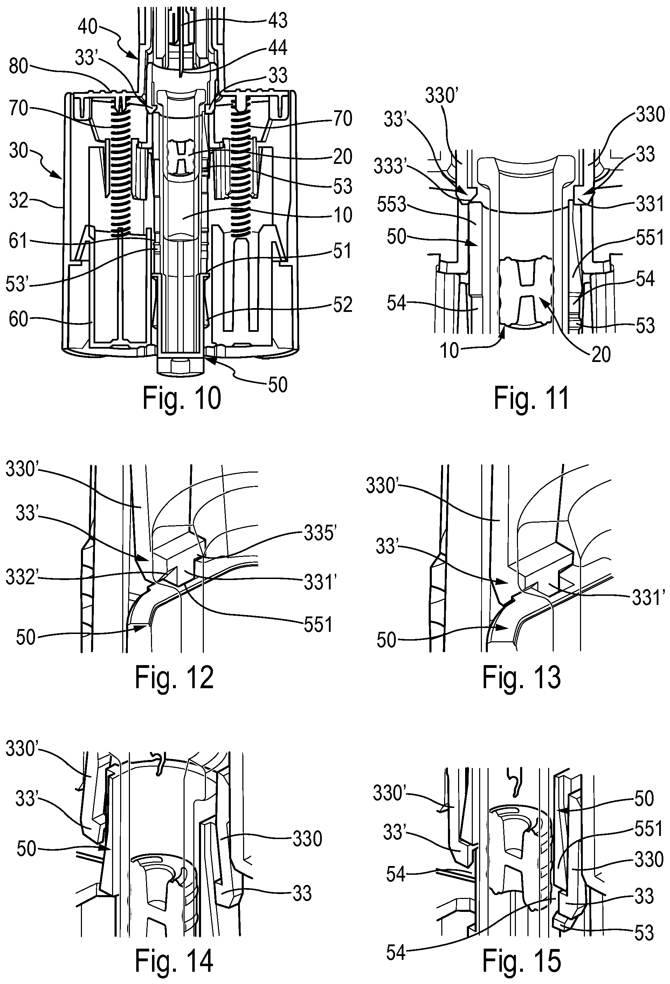

Ainsi, comme visible sur les figures 10 à 15, qui illustrent l'assemblage de l'organe de support 50 dans le corps principal 30, les zones inclinées 332, 333 et 332', 333' de chaque élément de poussée 33, 33' vont glisser sur le bord axial supérieur des nervures latérales 552, 553, ce qui va déformer les pattes déformables 330, 330' radialement vers l'extérieur. Chaque zone de poussée 331, 331' va donc éviter tout contact avec l'organe de support 50, en étant d'abord face à la partie de plus petit diamètre de chaque rampe centrale 551, puis en s'écartant radialement vers l'extérieur au fur et à mesure de l'assemblage.

En fin d'assemblage, visible sur la figure 15, les pattes déformables 330, 330' viennent s'encliqueter dans une première fenêtre 54 respective, avec donc le premier élément de poussée 33 disposé face au premier élément de résistance 53, ce qui permettra de réaliser l'accumulation d'énergie lors de la distribution de la première dose. La zone de poussée centrale 331 n'ayant subi aucun contact avec l'organe de support 50 lors de l'assemblage, cette accumulation d'énergie sera parfaitement conforme aux spécifications prévues.

Les seconds profils 55' étant identiques au premiers profils 55, il en est de même lors de la distribution de la première dose, de sorte qu'après cette première distribution, le second élément de poussée 33' est disposé face au second élément de résistance 53', ce qui permettra de réaliser l'accumulation d'énergie lors de la distribution de la seconde dose. La zone de poussée centrale 331' n'ayant subi aucun contact avec l'organe de support 50, ni lors de l'assemblage, ni lors de la distribution de la première dose, cette accumulation d'énergie sera parfaitement conforme aux spécifications prévues.

Bien entendu, la présente invention a été décrite en référence à deux modes de réalisation, qui ne sont pas limitatifs, et toute modification utile peut être apportée à la présente invention sans sortir du cadre de celle-ci tel que défini par les revendications annexées.

## Revendications

1. Dispositif de distribution de produit fluide comportant un réservoir (10) contenant deux doses de produit fluide, un organe de distribution (20), tel qu'un piston, monté coulissant dans ledit réservoir (10) pour distribuer du produit fluide, un corps principal (30), un corps central (90) solidaire dudit corps principal (30), un organe de support (50) recevant ledit réservoir (10), une tête de distribution (40) pourvue d'un orifice de distribution (41) et un organe d'actionnement (60) axialement déplaçable à l'intérieur dudit corps principal (30) et coopérant avec ledit organe de support (50) pour réaliser les actionnements successifs du dispositif en déplaçant ledit organe de support (50) par rapport audit corps central (90) pour ainsi déplacer ledit organe de distribution (20) dans ledit réservoir (10) et ainsi distribuer du produit fluide à travers ledit orifice de distribution (41), un ressort de rappel (70) étant prévu pour ramener ledit organe d'actionnement (60) dans sa position de départ après chaque actionnement, ledit dispositif comportant des moyens d'accumulation d'énergie comportant des premier et second éléments de poussée (33, 33'; 93, 93') formés sur ledit corps central (90) et des premier et second éléments de résistance (53, 53') formés sur ledit organe de support (50), ledit premier élément de poussée (33; 93) étant adapté à coopérer avec ledit premier élément de résistance (53) pour accumuler de l'énergie lors de la distribution de la première dose, et ledit second élément de poussée (33'; 93') étant adapté à coopérer avec ledit second élément de résistance (53') pour accumuler de l'énergie lors de la distribution de la seconde dose, chaque élément de poussée (33, 33'; 93, 93') comportant une patte radialement déformable (330, 330'; 930, 930'), pourvue à son extrémité axiale inférieure d'une zone de poussée centrale (331, 331'; 931, 931'), dont la surface axialement inférieure vient en contact d'un élément de résistance respectif (53, 53') lors des actionnements, **caractérisé en ce que**:
- ledit organe de support (50) comporte deux profils longitudinaux diamétralement opposés, chaque profil longitudinal comportant, en partant du bord axial supérieur : un premier profil de déformation (55), une première fenêtre (54), un second profil de déformation (55'), et une seconde fenêtre (54'),
- la première fenêtre (54) d'un profil longitudinal comporte ledit premier élément de résistance (53), et la seconde fenêtre (54') de l'autre profil longitudinal comporte ledit second élément de résistance (53'),
- chaque profil de déformation (55, 55') comporte : une rampe centrale (551, 551') s'étendant axialement et inclinée radialement vers l'extérieur en direction du bas, disposée entre des première et seconde nervures latérales (552, 552'; 553, 553') axialement droites et de dimension radiale constante,
- chaque zone de poussée centrale (331, 331'; 931, 931') est disposée entre une première et une seconde zone inclinée (332, 332' et 333, 333'; 932, 932' et 933, 933'),
- de telle sorte que lorsque ledit organe de support (50) se déplace axialement par rapport audit corps central (90) lors de l'assemblage, chaque élément de poussée (33, 33'; 93, 93') coopère avec un premier profil de déformation (55) respectif de telle sorte que chaque zone de poussée centrale (331, 331'; 931, 931') n'entre pas en contact avec ledit organe de support (50).

2. Dispositif selon la revendication 1, dans lequel lorsque ledit organe de support (50) se déplace axialement par rapport audit corps central (90) lors de la distribution de la première dose, ledit second élément de poussée (33'; 93') coopère avec un second profil de déformation (55') de telle sorte que sa zone de poussée centrale (331'; 931') n'entre pas en contact avec ledit organe de support (50).

3. Dispositif selon la revendication 1 ou 2, dans lequel lesdits premier et second éléments de résistance (53, 53') sont décalés axialement sur ledit corps de support (50).

4. Dispositif selon l'une quelconque des revendications précédentes, dans lequel lesdits premier et second éléments de poussée (33, 33'; 93, 93') sont disposés diamétralement opposés sur ledit corps central (90).

5. Dispositif selon l'une quelconque des revendications précédentes, dans lequel lesdits éléments de résistance (53, 53') sont formés par des ponts sécables, qui se cassent lors de l'actionnement, ou par des poutres déformables, qui se déforment lors de l'actionnement.

6. Dispositif selon l'une quelconque des revendications précédentes, dans lequel lesdits premier et second éléments de poussée (33, 93; 33', 93') sont identiques, ledit premier élément de poussée (33; 93) coopérant avec un profil longitudinal et ledit second élément de poussée (33'; 93') coopérant avec l'autre profil longitudinal.

7. Dispositif selon l'une quelconque des revendications précédentes, dans lequel lesdits premiers profils de déformation (55) coopèrent avec les deux éléments de poussée (33, 33'; 93, 93') lors de l'assemblage, et lesdits seconds profils de déformation (55') coopèrent avec lesdits deux éléments de poussée (33, 33'; 93, 93') lors de la distribution de la première dose.

8. Dispositif selon l'une quelconque des revendications précédentes, dans lequel la surface radialement interne (335) de chaque élément de poussée (33, 33'; 93, 93') a une forme de T.

9. Dispositif selon l'une quelconque des revendications précédentes, dans lequel, lors de l'assemblage, lesdites zones de poussée (331, 331'; 931, 931') vont chacune coopérer avec une rampe centrale respective (551) d'un premier profil de déformation (55), pendant que lesdites zones inclinées (332, 333; 932, 933 et 332', 333'; 932', 933') vont coopérer avec lesdites nervures latérales (552, 553).

10. Dispositif selon l'une quelconque des revendications précédentes, dans lequel, lors de la distribution de la première dose, lesdites zones de poussée (331, 331'; 931, 931') vont chacune coopérer avec une rampe centrale respective (551') d'un second profil de déformation (55'), pendant que lesdites zones inclinées (332, 333; 932, 933 et 332', 333'; 932', 933') vont coopérer avec lesdites nervures latérales (552', 553').

11. Dispositif selon l'une quelconque des revendications précédentes, dans lequel, lors de l'assemblage, lesdites zones inclinées (332, 333; 332', 333') de chaque élément de poussée (33, 33'; 93, 93') glissent sur le bord axial supérieur desdites nervures latérales (552, 553) dudit premier profil de déformation (55), ce qui déforme lesdites pattes déformables (330, 330'; 930, 930') radialement vers l'extérieur, de sorte que chaque zone de poussée (331, 331'; 931, 931') évite tout contact avec ledit organe de support (50), en étant d'abord face à la partie de plus petit diamètre de chaque rampe centrale (551), puis en s'écartant radialement vers l'extérieur au fur et à mesure de l'assemblage.

12. Dispositif selon l'une quelconque des revendications précédentes, dans lequel, lors de la distribution de la première dose, lesdites zones inclinées (332, 333; 332', 333') de chaque élément de poussée (33, 33'; 93, 93') glissent sur le bord axial supérieur desdites nervures latérales (552', 553') dudit second profil de déformation (55'), ce qui déforme lesdites pattes déformables (330, 330'; 930, 930') radialement vers l'extérieur, de sorte que chaque zone de poussée (331, 331'; 931, 931') évite tout contact avec ledit organe de support (50), en étant d'abord face à la partie de plus petit diamètre de chaque rampe centrale (551'), puis en s'écartant radialement vers l'extérieur au fur et à mesure de la distribution de la première dose.

13. Dispositif selon l'une quelconque des revendications précédentes, dans lequel, en fin d'assemblage, lesdites pattes déformables (330, 330'; 930, 930') s'encliquètent dans une première fenêtre (54) respective, ledit premier élément de poussée (33, 93) étant disposé face audit premier élément de résistance (53), pour réaliser l'accumulation d'énergie lors de la distribution de la première dose.

14. Dispositif selon l'une quelconque des revendications précédentes, dans lequel, en fin de distribution de la première dose, lesdites pattes déformables (330, 330'; 930, 930') s'encliquètent dans une seconde fenêtre (54') respective, ledit second élément de poussée (33', 93') étant disposé face audit second élément de résistance (53'), pour réaliser l'accumulation d'énergie lors de la distribution de la seconde dose.

## Patentansprüche

1. Vorrichtung zur Abgabe eines fluiden Produkts, aufweisend einen Behälter (10), der zwei Dosen des fluiden Produkts enthält, ein Abgabeelement (20) wie ein Kolben, der in dem Behälter (10) gleitend angebracht ist, um das fluide Produkt abzugeben, einen Hauptkörper (30), einen mit dem Hauptkörper (30) fest verbundenen Mittelkörper (90), ein den Behälter (10) aufnehmendes Halteelement (50), einen mit einer Abgabeöffnung **(41)** versehenen Abgabekopf (40) und ein axial innerhalb des Hauptkörpers (30) verlagerbares und mit dem Halteelement (50) zusammenwirkendes Betätigungselement (60) zur Durchführung von aufeinanderfolgenden Betätigungen der Vorrichtung durch Verlagern des Halteelements (50) relativ zu dem Mittelkörper (90), um somit das Abgabeelement (20) in dem Behälter (10) zu verlagern und somit fluides Produkt durch die Abgabeöffnung (41) abzugeben, wobei eine Rückstellfeder (70) vorgesehen ist, um das Betätigungselement (60) nach jeder Betätigung in seine Ausgangsposition zurückzuführen, wobei die Vorrichtung Energiespeichermittel aufweist, die ein erstes und ein zweites Druckelement (33, 33'; 93, 93') aufweist, die an dem Mittelkörper (90) ausgebildet sind, sowie ein erstes und ein zweites Widerstandselement (53, 53'), die an dem Halteelement (50) ausgebildet sind, wobei das erste Druckelement (33; 93) geeignet ist, mit dem ersten Widerstandselement (53) zusammenzuwirken, um bei der Abgabe der ersten Dosis Energie zu speichern, und das zweite Druckelement (33'; 93') geeignet ist, mit dem zweiten Widerstandselement (53') zusammenzuwirken, um bei der Abgabe der zweiten Dosis Energie zu speichern, wobei jedes Druckelement (33, 33'; 93, 93') eine radial verformbare Lasche (330, 330'; 930, 930') aufweist, die an ihrem unteren axialen Ende mit einem mittleren Druckbereich (331, 331'; 931, 931') versehen ist, dessen axial untere Fläche bei Betätigungen mit einem jeweiligen Widerstandselement (53, 53') in Kontakt kommt, **dadurch gekennzeichnet, dass**:
- das Halteelement (50) zwei diametral gegenüberliegende Längsprofile aufweist, wobei jedes Längsprofil, ausgehend von der axialen Oberkante, aufweist: ein erstes Verformungsprofil (55), ein erstes Fenster (54), ein zweites Verformungsprofil (55') und ein zweites Fenster (54'),
- das erste Fenster (54) eines Längsprofils das erste Widerstandselement (53) aufweist und das zweite Fenster (54') des anderen Längsprofils das zweite Widerstandselement (53') aufweist,
- jedes Verformungsprofil (55, 55') aufweist: eine mittlere Rampe (551, 551'), die sich axial erstreckt und radial nach außen in Richtung unten geneigt und zwischen einer ersten und einer zweiten axial geraden Seitenrippe (552, 552'; 553, 553') mit konstanter radialer Abmessung angeordnet ist,
- jeder mittlere Druckbereich (331, 331'; 931, 931') zwischen einem ersten und einem zweiten geneigten Bereich (332, 332' und 333, 333'; 932, 932' und 933, 933') angeordnet ist,
- so dass, wenn sich das Halteelement (50) beim Zusammenbau axial relativ zum Mittelkörper (90) verlagert, jedes Druckelement (33, 33'; 93, 93') mit einem jeweiligen ersten Verformungsprofil (55) derart zusammenwirkt, dass jeder mittlere Druckbereich (331, 331'; 931, 931') nicht mit dem Halteelement (50) in Kontakt kommt.

2. Vorrichtung nach Anspruch 1, wobei, wenn sich das Halteelement (50) bei der Abgabe der ersten Dosis axial relativ zum Mittelkörper (90) verlagert, das zweite Druckelement (33'; 93') mit einem zweiten Verformungsprofil (55') derart zusammenwirkt, dass sein mittlerer Druckbereich (331'; 931') nicht mit dem Halteelement (50) in Kontakt kommt.

3. Vorrichtung nach Anspruch 1 oder 2, wobei das erste und das zweite Widerstandselement (53, 53') auf dem Haltekörper (50) axial versetzt sind.

4. Vorrichtung nach einem der vorstehenden Ansprüche, wobei das erste und das zweite Druckelement (33, 33'; 93, 93') diametral gegenüberliegend auf dem Mittelkörper (90) angeordnet sind.

5. Vorrichtung nach einem der vorstehenden Ansprüche, wobei die Widerstandselemente (53, 53') durch brechbare Brücken, die bei Betätigung brechen, oder durch verformbare Träger, die sich bei Betätigung verformen, gebildet sind.

6. Vorrichtung nach einem der vorstehenden Ansprüche, wobei das erste und das zweite Druckelement (33, 93; 33', 93') identisch sind, wobei das erste Druckelement (33; 93) mit einem Längsprofil zusammenwirkt und das zweite Druckelement (33'; 93') mit dem anderen Längsprofil zusammenwirkt.

7. Vorrichtung nach einem der vorstehenden Ansprüche, wobei die ersten Verformungsprofile (55) beim Zusammenbau mit den beiden Druckelementen (33, 33'; 93, 93') zusammenwirken und die zweiten Verformungsprofile (55') bei der Abgabe der ersten Dosis mit den beiden Druckelementen (33, 33'; 93, 93') zusammenwirken.

8. Vorrichtung nach einem der vorstehenden Ansprüche, wobei die radial innere Fläche (335) jedes Druckelements (33, 33'; 93, 93') eine T-Form aufweist.

9. Vorrichtung nach einem der vorstehenden Ansprüche, wobei die Druckbereiche (331, 331'; 931, 931') beim Zusammenbau jeweils mit einer entsprechenden mittleren Rampe (551) eines ersten Verformungsprofils (55) zusammenwirken, während die geneigten Bereiche (332, 333; 932, 933 und 332', 333'; 932', 933') mit den Seitenrippen (552, 553) zusammenwirken.

10. Vorrichtung nach einem der vorstehenden Ansprüche, wobei bei der Abgabe der ersten Dosis die Druckbereiche (331, 331'; 931, 931') jeweils mit einer entsprechenden mittleren Rampe (551') eines zweiten Verformungsprofils (55') zusammenwirken, während die geneigten Bereiche (332, 333; 932, 933 und 332', 333'; 932', 933') mit den Seitenrippen (552', 553') zusammenwirken.

11. Vorrichtung nach einem der vorstehenden Ansprüche, wobei beim Zusammenbau die geneigten Bereiche (332, 333; 332', 333') jedes Druckelements (33, 33'; 93, 93') über die obere axiale Kante der Seitenrippen (552, 553) des ersten Verformungsprofils (55) gleiten, wodurch die verformbaren Laschen (330, 330'; 930, 930') radial nach außen verformt werden, so dass jeder Druckbereich (331, 331'; 931, 931') jeglichen Kontakt mit dem Halteelement (50) vermeidet, indem er zunächst dem Teil mit dem kleineren Durchmesser jeder mittleren Rampe (551) gegenüberliegt und sich dann im Laufe des Zusammenbaus radial nach außen entfernt.

12. Vorrichtung nach einem der vorstehenden Ansprüche, wobei bei der Abgabe der ersten Dosis die geneigten Bereiche (332, 333; 332', 333') jedes Druckelements (33, 33'; 93, 93') über die obere axiale Kante der Seitenrippen (552', 553') des zweiten Verformungsprofils (55') gleiten, wodurch die verformbaren Laschen (330, 330'; 930, 930') radial nach außen verformt werden, so dass jeder Druckbereich (331, 331'; 931, 931') jeglichen Kontakt mit dem Halteelement (50) vermeidet, indem er zunächst dem Teil mit dem kleineren Durchmesser jeder mittleren Rampe (551') gegenüberliegt und sich dann während der Abgabe der ersten Dosis radial nach außen entfernt.

13. Vorrichtung nach einem der vorstehenden Ansprüche, wobei am Ende des Zusammenbaus die verformbaren Laschen (330, 330'; 930, 930') in ein jeweiliges erstes Fenster (54) einrasten, wobei das erste Druckelement (33, 93) gegenüber dem ersten Widerstandselement (53) angeordnet ist, um bei der Abgabe der ersten Dosis Energie zu speichern.

14. Vorrichtung nach einem der vorstehenden Ansprüche, wobei am Ende der Abgabe der ersten Dosis die verformbaren Laschen (330, 330'; 930, 930') in ein jeweiliges zweites Fenster (54') einrasten, wobei das zweite Druckelement (33', 93') gegenüber dem zweiten Widerstandselement (53') angeordnet ist, um bei der Abgabe der zweiten Dosis Energie zu speichern.

## Claims

1. Device for dispensing a fluid product comprising a reservoir (10) containing two doses of fluid product, a dispensing member (20), such as a piston, slidingly mounted in said reservoir (10) to dispense a fluid product, a main body (30), a central body (90) secured to said main body (30), a support member (50) that accommodates said reservoir (10), a dispensing head (40) provided with a dispensing orifice (41) and an actuating member (60) that is axially displaceable within said main body (30) and engages with said support member (50) to perform successive actuations of the device by displacing said support member (50) with respect to said central body (90) to thus displace said dispensing member (20) in said reservoir (10) and thus dispense the fluid product through said dispensing orifice (41), a return spring (70) being provided to return said actuating member (60) into its starting position after each actuation, said device comprising energy accumulation means comprising first and second pushing elements (33, 33'; 93, 93') formed on said central body (90) and first and second resistance elements (53, 53') formed on said support member (50), said first pushing element (33; 93) being suitable for engaging with said first resistance element (53) to accumulate energy during the dispensing of the first dose, and said second pushing element (33'; 93') being suitable for engaging with said second resistance element (53') to accumulate energy during the dispensing of the second dose, each pushing element (33, 33'; 93, 93') comprising a radially deformable tab (330, 330'; 930, 930'), provided at its lower axial end with a central pushing zone (331, 331'; 931, 931'), the axially lower surface of which comes into contact with a respective resistance element (53, 53') during actuations, **characterised in that**:
- said support member (50) comprises two diametrically opposite longitudinal profiles, each longitudinal profile comprising, starting with the upper axial edge: a first deformation profile (55), a first window (54), a second deformation profile (55'), and a second window (54'),
- the first window (54) of a longitudinal profile comprises said first resistance element (53), and the second window (54') of the other longitudinal profile comprises said second resistance element (53'),
- each deformation profile (55, 55') comprises: a central ramp (551, 551') extending axially and radially inclined outwards towards the bottom, disposed between first and second side ridges (552, 552'; 553, 553') axially straight and with a constant radial dimension,
- each central pushing zone (331, 331'; 931, 931') is disposed between a first and a second inclined zone (332, 332' and 333, 333'; 932, 932' and 933, 933'),
- such that when said support member (50) is axially displaced with respect to said central body (90) during assembly, each pushing element (33, 33'; 93, 93') engages with a respective first deformation profile (55), such that each central pushing zone (331, 331'; 931, 931') does not come into contact with said support member (50).

2. Device according to claim 1, wherein when said support member (50) is axially displaced with respect to said central body (90) during the dispensing of the first dose, said second pushing element (33'; 93') engages with a second deformation profile (55'), such that its central pushing zone (331'; 931') does not come into contact with said support member (50).

3. Device according to claim 1 or 2, wherein said first and second resistance elements (53, 53') are axially offset on said support body (50).

4. Device according to any one of the preceding claims, wherein said first and second pushing elements (33, 33'; 93, 93') are disposed diametrically opposite on said central body (90).

5. Device according to any one of the preceding claims, wherein said resistance elements (53, 53') are formed by breakable bridges, which are broken during actuation, or by deformable beams, which are deformed during actuation.

6. Device according to any one of the preceding claims, wherein said first and second pushing elements (33, 93; 33', 93') are identical, said first pushing element (33; 93) engaging with a longitudinal profile and said second pushing element (33'; 93') engaging with the other longitudinal profile.

7. Device according to any one of the preceding claims, wherein said first deformation profiles (55) engage with the two pushing elements (33, 33'; 93, 93') during assembly, and said second deformation profiles (55') engage with said two pushing elements (33, 33'; 93, 93') during the dispensing of the first dose.

8. Device according to any one of the preceding claims, wherein the radially internal surface (335) of each pushing element (33, 33'; 93, 93') is T-shaped.

9. Device according to any one of the preceding claims, wherein, during assembly, said pushing zones (331, 331'; 931, 931') will each engage with a respective central ramp (551) of a first deformation profile (55), while said inclined zones (332, 333; 932, 933 and 332', 333'; 932', 933') will engage with said side ridges (552, 553).

10. Device according to any one of the preceding claims, wherein, during the dispensing of the first dose, said pushing zones (331, 331'; 931, 931') will each engage with a respective central ramp (551') of a second deformation profile (55'), while said inclined zones (332, 333; 932, 933 and 332', 333'; 932', 933') will engage with said side ridges (552', 553').

11. Device according to any one of the preceding claims, wherein, during assembly, said inclined zones (332, 333; 332', 333') of each pushing element (33, 33'; 93, 93') slide over the upper axial edge of said side ridges (552, 553) of said first deformation profile (55), which deforms said deformable tabs (330, 330'; 930, 930') radially outwards, such that each pushing zone (331, 331'; 931, 931') avoids any contact with said support member (50), by first facing the part with the smallest diameter of each central ramp (551), then by moving away radially outwards according to the assembly.

12. Device according to any one of the preceding claims, wherein, during the dispensing of the first dose, said inclined zones (332, 333; 332', 333') of each pushing element (33, 33'; 93, 93') slide over the upper axial edge of said side ridges (552', 553') of said second deformation profile (55'), which deforms said deformable tabs (330, 330'; 930, 930') radially outwards, such that each pushing zone (331, 331'; 931, 931') avoids any contact with said support member (50), by first facing the part with the smallest diameter of each central ramp (551'), then by moving away radially outwards according to the dispensing of the first dose.

13. Device according to any one of the preceding claims, wherein, at the end of assembly, said deformable tabs (330, 330'; 930, 930') are snap-fitted in a respective first window (54), said first pushing element (33, 93) being disposed facing said first resistance element (53), to store energy during the dispensing of the first dose.

14. Device according to any one of the preceding claims, wherein, at the end of dispensing of the first dose, said deformable tabs (330, 330'; 930, 930') are snap-fitted in a respective second window (54'), said second pushing element (33', 93') being disposed facing said second resistance element (53'), to store energy during the dispensing of the second dose.
